# EUROPEAN PATENT SPECIFICATION

(11) **EP 3 824 297 B1**
(45) Date of publication and mention of the grant of the patent: **21.02.2024**
(21) Application number: 19894808.5
(22) Date of filing: 06.05.2019
(51) Int. Cl.: G01N 35/08, B01L 7/00, B01L 3/00

(54) **RAPID THERMAL CYCLING DEVICES**
SCHNELLE WÄRMEKREISLAUFVORRICHTUNG
DISPOSITIFS DE CYCLAGE THERMIQUE RAPIDE

(30) Priority: 13.12.2018 WO PCT/US2018/065498
(43) Date of publication of application: 26.05.2021
(73) Proprietor: Hewlett-Packard Development Company, L.P., Spring TX 77389 (US)
(72) Inventor: KORNILOVICH, Pavel, Corvallis, Oregon 97330-4241 (US); GOVYADINOV, Alexander, Corvallis, Oregon 97330-4241 (US)
(74) Representative: Haseltine Lake Kempner LLP
(86) International application number: PCT/US2019/030830
(87) International publication number: WO 2020/122979

(56) References cited:
- WO-A1-2014/025924
- CN-A- 107 603 874
- US-A1- 2002 022 261
- US-A1- 2008 064 086
- US-A1- 2014 030 766
- US-A1- 2015 031 573
- US-A1- 2015 321 195

## Description

### BACKGROUND

Nucleic acid amplification is a technique utilized in research, medical diagnostics, pathogen detection, and forensic testing. The ability to amplify a small quantity of a sample of a nucleic acid to generate copies of the nucleic acid in the sample can permit research, medical diagnostic, pathogen detection, and forensic tests that would not otherwise be practical due to the small quantity of the sample, for example. Further background art is described in WO 2014/025924 A1 and US 2002/022261 A1.

### BRIEF DESCRIPTION OF THE DRAWINGS

FIG. 1A schematically illustrates a cross-sectional view of an example rapid thermal cycling device in accordance with the present invention
FIG. 1B schematically illustrates a cross-sectional view of an example rapid thermal cycling device in accordance with the present invention
FIG. 2 schematically illustrates a cross-sectional view of an example rapid thermal cycling device in accordance with the present invention
FIG. 3 schematically illustrates a top view of an example rapid thermal cycling device in accordance with the present invention
FIG. 4 schematically illustrates a cross-sectional view of an example rapid thermal cycling system in accordance with the present invention
FIG. 5 is a flow diagram illustrating an example method of manufacturing a rapid thermal cycling device in an example use in accordance with the present invention
FIG. 6 schematically illustrates an example method of manufacturing a rapid thermal cycling device in an example use in accordance with the present invention and
FIG. 7 schematically illustrates an example method of manufacturing a rapid thermal cycling device in an example use in accordance with the present invention

### DETAILED DESCRIPTION

Nucleic acid amplification can include denaturing, annealing, and extending nucleic acid chains. Typically, the amplification process utilizes specialized equipment that can be costly and cumbersome, and in some instances a thermal cycle may vary from 1 minute to 2 minutes and the amplification process can take around an hour, e.g., 25 to 45 thermal cycles, or 35 to 40 thermal cycles. During denaturing an increased temperature can cause hydrogen bonds between bases in a double stranded nucleic acid sample to break apart resulting in two single strands realized from a formerly double stranded nucleic acid. During annealing, the heated sample can then be cooled, enabling single stranded nucleic acid oligomers, such as primers, to attach to the complementary nitrogen bases on the single strands of the nucleic acid. During extending of the nucleic acid chain the temperature may be increased, for example, to enable a polymerase enzyme to extend the nucleic acid strand by adding nucleic acid bases. Regardless of the sequence or heating and cooling and the temperatures that are reached during the heating and cooling phases, once the temperature cycling profile is established or approximated, this thermal cycling can be repeated until a desired number of nucleic acid copies, e.g., DNA, are formed, which can for example take from about 10 to about 100 thermal cycles, or 20 to 60 thermal cycles in many instances.

A "thermal cycle" can be based on a range of temperatures used for denaturing, annealing, and extending phases of the amplification, with temperatures raising and lowering for various stages within the cycle. For simplification, a thermal cycle can be defined as a series of temperatures within a range of temperatures defined by the uppermost temperature and a lowermost temperature. The cycle can be determined based on either the uppermost or lowermost temperature of the series of temperatures. For example, if a thermal cycle repeated the following temperatures: 75°C heated to 95°C cooled to 45°C heated to 98°C cooled to initial temperature for the amplification, then the thermal cycle can be counted based on the phase where the temperatures reaches about 98 °C, with the first occurrence defining the last temperature of cycle 1. The same could be said of 45 °C, which can also be used as the event that occurs to count or identify the cycle. Regardless of temperatures reached between the two endpoint temperatures, generally the temperature can oscillate between a maximum temperature and a minimum temperature, e.g., general heating and cooling stages of the cycle. However, in another example, the thermal profile can be more continuous, within the timeframes outlined herein, or even without specific set timeframes due to the thermal mass of the device and nucleic acid sample fluid and temperature tolerance for reaction phase. In addition, there are some examples where annealing and extension can be carried out at about the same temperature, which may allow for the device to be used outside of the thermal cycling profiles described generally herein. Still further, in some examples, the amplification or other similar cycling can also be modulated in real time, which often can correspond to modulation of the thermal cycling. Furthermore, there can also be modifications to the thermal cycling during various phases of the processing of the nucleic acid sample fluid, such as modification of an initial phase prior to starting the first amplification cycle. For example, a reverse transcription (RT) process and/or introduction of hot-start PCR enzyme could occur during an initial step. In still further detail, the present disclosure can also provide for isothermal amplification using enzymes supporting processes such as loop-mediated isothermal amplification (LAMP) or recombinase polymerase amplification (RPA).

In accordance with the present invention a rapid thermal cycling device includes a microfluidic reaction chamber, a dry reagent, and a heating element. The microfluidic reaction chamber is defined between a substrate and a cover having an average space therebetween from 10 µm to 150 µm. The dry reagent is positioned within the microfluidic reaction chamber. The heating element is immediately adjacent and thermally coupled to the microfluidic reaction chamber to heat a fluid when introduced therein. The substrate includes a thermal diffusing layer having a thermal conductivity that ranges from 20 W/m/K to 2,000 W/m/K as measured at PCR temperatures. As used herein PCR, relevant temperatures can be from about 40°C to 100°C, for example. The thermal diffusing layer has a thickness that is 3 to 40 times greater than the average space between the substrate and the cover. In one example, the substrate further includes a thermal stabilizing layer that is positioned and operable as a heat sink for the thermal diffusing layer. In a further example, the microfluidic chamber includes nucleic acid primers conjugated thereto. In one example, the dry reagent includes thermostable nucleic acid polymerase, dNTPs, PCR primers, magnesium salt, or a combination thereof. In another example, the heating element is thermally coupled the microfluidic reaction chamber to heat fluid in the microfluidic reaction chamber at a rate of 100 °C/s to 50,000,000 °C/s. The heating element includes a heating interface surface that is dimensionally as large or larger in surface area as a microfluidic reaction chamber interface area where the substrate defines the microfluidic reaction chamber. In yet another example, the rapid thermal cycling device further includes a plurality of secondary microfluidic reaction chambers arranged fluidically in parallel with respect to the microfluidic reaction chamber. A sample input microchannel commonly feeds the microfluidic reaction chamber and the plurality of secondary microfluidic reaction chambers. In one example, a ratio of a width of the microfluidic reaction chamber to a length of the sample input microchannel ranges from 1:3 to 1:100. In another example, the microfluidic channel has a serpentine configuration. In yet another example, the microfluidic reaction chamber is included as part of an on-chip, internally controlled, device.

In another example, a rapid thermal cycling system is presented. The rapid thermal cycling system includes a rapid thermal cycling device and a detection device. The rapid thermal cycling device includes a microfluidic reaction chamber, a dry reagent, and a heating element. The microfluidic reaction chamber is defined between a substrate and a cover having an average space therebetween from 4 µm to 150 µm. The dry reagent is positioned within the microfluidic reaction chamber. The heating element is thermally coupled to the microfluidic reaction chamber to heat a fluid when introduced therein. The detection device is coupled to the microfluidic reaction chamber to receive data related to fluid prior to, during, or after heat cycling the fluid within the microfluidic reaction chamber. In one example, the detection device includes a single-color illumination and detection imaging system, multi-color illumination and detection imaging system, an electrochemical detection system, an optical photodiode, or a combination thereof.

In another aspect of the invention, a method of manufacturing a rapid thermal cycling device includes loading a reagent on a substrate, air drying or freeze-drying the reagent on the substrate to form a dry reagent, e.g., lyophilization, and forming a microfluidic reaction chamber including a sample input port. The microfluidic reaction chamber is formed about the reagent or the dry reagent and has an average height from 4 µm to 150 µm. In one example, the method further includes forming the microfluidic reaction chamber by applying a cover onto the substrate to leave the microfluidic reaction chamber therebetween. The forming of the microfluidic reaction chamber occurs either prior to or after a lyophilization process.

When discussing the rapid thermal cycling device, the rapid thermal cycling system, and/or the method of manufacturing a rapid thermal cycling device herein, these discussions can be considered applicable to one another whether or not they are explicitly discussed in the context of that example. Thus, for example, when discussing a microfluidic reaction chamber related to a rapid thermal cycling device, such disclosure is also relevant to and directly supported in the context of the rapid thermal cycling system, the method of manufacturing a rapid thermal cycling device, and *vice versa.* Furthermore, terms used herein will take on the ordinary meaning in the relevant technical field unless specified otherwise. In some instances, there are terms defined more specifically throughout the specification or included at the end of the present specification, and thus, these terms can have a meaning as described herein.

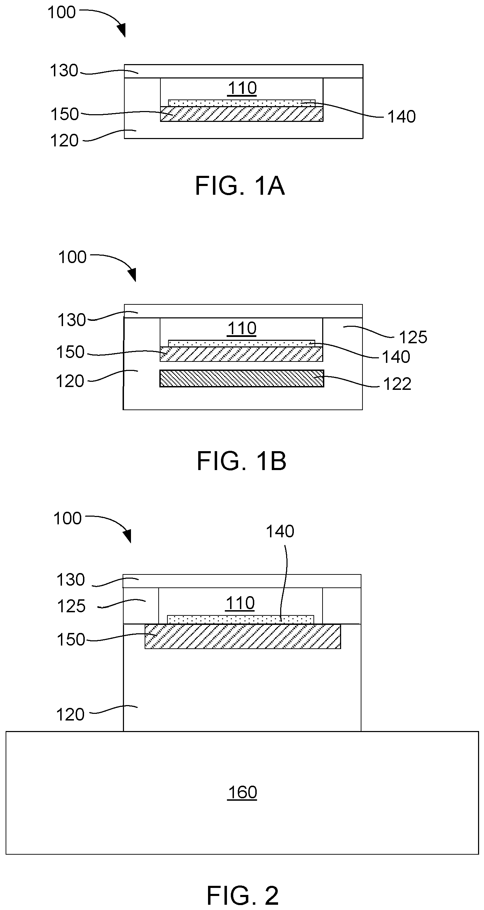

The rapid thermal cycling device 100, as shown by different examples in a cross-sectional view in FIG. 1A, FIG. 1B, and FIG. 2, can include a microfluidic reaction chamber 110, which in one example can be defined between a substrate 120 and a cover 130 or lid. In this example, the walls are shown as being constructed from the substrate, but in some examples the walls can be of a different material, e.g., provided by the lid (not shown) or by a separate structural wall of a different material (as shown in FIG. 2). For example, in some examples, the substrate can serve as a thermally diffusive layer, e.g., silicon, while the walls can be made from a materials such as SU8 or other material. The average space between the substrate (from the base thereof, e.g., opposite the cover) and the cover can be from 4 µm to 150 µm. A heating element 150 can be thermally coupled to the microfluidic reaction chamber so that it can heat a fluid (not shown) when present therein. The heating element is immediately adjacent to the microfluidic reaction chamber. In an alternative configuration falling outside the scope of the present invention, the heating element can be carried within the substrate and spatially separated (but still thermally coupled) to the microfluidic reaction chamber. A dry reagent 140, is also be positioned within the microfluidic reaction chamber.

In further detail, a material of the substrate 120 can vary. According to the invention, the material of the substrate includes a thermally diffusive material having a thermal conductivity that ranges from 20 W/m/K to 2,000 W/m/K, as shown in FIG. 1A.

Additionally, there can be a separate thermally diffusive material layer having a thermal conductivity that ranges from 20 W/m/K to 2,000 W/m/K, as shown at layer thermally diffusive layer 122 in FIG. 1B. The thermally diffusive material of the substrate and/or the separate thermally diffusive layer (if present) can alternatively have, for example, a thermal conductivity that ranges from 100 W/m/K to 1,000 W/m/K, from 50 W/m/K to 500 W/m/K, or from 1,000 W/m/K to 2,000 W/m/K. The thermally diffusive materials that can be used include silicon, silicon nitride, polycrystalline silicon dioxide, pyrolytic graphite, aluminum, copper, or a combination thereof. In one example, the thermally diffusive material can include silicon.

The thermally diffusive material 120 of the substrate 120 or thermally diffusive layer 122 can act as a heat sink, thereby permitting a fluid when present in the reaction chamber 110 to cool quickly without external cooling or other assistive cooling elements. In one example, the thermally diffusive material can be a passive thermally diffusive material and can rapidly dissipate or remove heat from the microfluidic reaction chamber without input from an active external device or chemical, such as a cooling device, coolant, a fan, another mechanical cooler, or the like. Thus, a passive thermally diffusive material does not rely on an active mechanism to transfer thermal energy from a higher temperature area away to a lower temperature area. In some examples, the heat transfer can occur from a fluid within the microfluidic reaction chamber to the substrate, and more particularly the thermally diffusive layer, which can contribute to heat diffusion from the microfluidic chamber.

A thickness of the thermally diffusive material can contribute to the material's ability to propagate heat through the material. A thicker substrate 120 or thermally diffusive layer 122 of material can propagate more heat than a thinner layer of material of the same material. According to the invention, a thickness of the thermally diffusive material is greater than the average space between the substrate and the cover 130 such that the thermally diffusive material can have greater potential to diffuse heat from a fluid in the space therebetween. Specifically, the thermal diffusing material has a thickness 3 to 40 times greater than the average space between the substrate and the cover. For example, the thermal diffusing material can have a thickness that can be from 15 to 35 times greater, from 25 to 40 times greater, from 10 to 30 times greater, or from 5 to 30 times greater than the average space between the substrate and the cover. In some examples, a thickness of the thermally diffusive material, e.g., substrate or layer, can range from 400 µm to 800 µm, from 500 µm to 700 µm, or from 550 µm to 750 µm.

In some examples, the substrate 120 can further be associated with or include a thermal stabilizing layer 160 that can be positioned to function as a heat sink for the thermal diffusing material (substrate and/or layer). See FIG. 2. In one example, a thermal stabilizing layer can have a thermal conductivity that can range from 10W/m/K to 2000 W/m/K when measured at 373°K (100 °C) and 10⁵ Pa. Example of thermally diffusive materials that can be used include ceramics and/or metal materials, such as alumina ceramic, aluminum, aluminum alloy, aluminum oxide, hafnia, hafnia alloy, zirconia, zirconia alloy, silver, silver alloy, gold, gold alloy, copper, copper alloy, tin, tin alloy, iron, iron alloy, or a combination thereof. In one example, the thermal stabilizing layer can be a layer constructed of alumina ceramic. In another example, the thermal stabilizing layer can include hafnia.

A thickness of the thermal stabilizing layer can further contribute to the layers potential to propagate heat from the thermally diffusive layer and to function as a heat sink for the thermally diffusive layer. In one example, the thermal stabilizing layer can have a thickness that is 1 mm to 10 mm thick. In yet other examples, the thermal stabilizing layer can have a thickness that can be from 3 mm to 7 mm, from 2 mm to 8 mm, from 4 mm to 6 mm, from 1 mm to 8 mm, or from 2 mm to 6 mm. In some examples, the thermal stabilizing layer can have a surface area at an interface with the thermally diffusive layer that can be as large or larger.

A cover 130 encloses the microfluidic reaction chamber 110 and can define an "upper" surface or ceiling of the microfluidic reaction chamber (as shown in FIG. 1A, FIG. 1B, and FIG. 2), or can define the upper surface/ceiling and a plurality of side walls 125 of the microfluidic reaction chamber. It is noted that when referring to upper surfaces, ceilings, side walls, floors, etc., with respect to the microfluidic reaction chamber, these terms are used for convenience as the chamber can be in any orientation, with the "ceiling" facing sideways or downward. Thus, these terms are to be defined as being relative to one another, and not to define the ultimate orientation of the chamber when loading or in use. With this understanding, the cover can be a removable closure for loading a fluid sample into the device or a fluid sample can be loaded into the device by microfluidic channels with the cover being a more permanently attached structure defined as a "cover" for convenience. In another example, the cover can be affixed to walls of the substrate 120. In other examples, the cover can include an opening configured to permit loading of a fluid into the static microfluidic reaction chamber.

The cover 130 can include a material such as glass, coverslip tape, silicon, SU8, or a combination thereof. In one example, the cover can include glass. In another example, the cover can include a coverslip tape. In some examples, the cover can be used to prevent moisture from entering the microfluidic reaction chamber 110 and hydrolyzing the dry reagent. In yet other examples, the cover can be a thermal diffusion cover that can contribute to heat diffusion from a fluid in the static microfluidic reaction chamber. Thermal diffusion covers can include a heat diffusing material as described above.

The microfluidic reaction chamber 110 includes a cavity defined by the substrate 120 and the cover 130, with side walls 125 provided by either the substrate, the cover, or as separate standoff structure(s) positioned between the substrate and the cover. The walls of the microfluidic reaction chamber can be composed of silicon, silicon dioxide, SU8, PDMS, or a combination thereof. In one example, the substrate can include silicon and the walls can include SU8. In a further example, a wall of the microfluidic chamber can have nucleic acid primers conjugated thereto, or there can be other structures that may be associated with the nucleic acid primers.

A depth of the microfluidic reaction chamber 110 (vertical height, again without regard to orientation but as a relative measurement between a base of substrate 120 and the cover 130 typically facing one another) can contribute to a rate at which a fluid when loaded in the microfluidic reaction chamber can heat and cool. According to the invention, the microfluidic reaction chamber has a depth ranging from from 4 µm to 150 µm. For example, the static microfluidic reaction chamber can have a depth that can range from 10 µm to 100 µm, from 30 µm to 90 µm, from 25 µm to 75 µm, 20 µm to 50 µm, 10 µm to 60 µm, or from 10 µm to 20 µm. In one example, the microfluidic reaction chamber can receive from 1 µL to 10 µL of fluid. In other examples, the microfluidic reaction chamber can receive from 10 pL to 10 µL of fluid, or from 50 pL to 200 pL of fluid. The quantity of fluid that can be loaded in the microfluidic reaction chamber can be increased by increasing an area of the interface between the microfluidic reaction chamber and the heating element 150 or the substrate, rather than increasing the depth of the microfluidic reaction chamber. In one example, the reaction chamber volume can be increased to 10 nL to 100 nL by increasing the area of the interface between the microfluidic reaction chamber and the heating element.

A dry reagent 140 is positioned in the cavity of the microfluidic reaction chamber 110. In one example, the dry reagent can be air dried or freeze-dried PCR master-mix. In another example, the dry reagent can include a thermostable nucleic acid polymerase, DNTPs, PCR primers, magnesium salt, or a combination thereof. In yet another example, the dry reagent can include a thermostable nucleic acid polymerase and magnesium salt. In a further example, the dry reagent can include polymerase enzymes, e.g., Taq polymerase enzyme; buffering components; organic cosolvents; minerals; other liquid vehicle components, etc. that can be air dried or freeze-dried within the microfluidic reaction chamber. The dry reagent can be in powdered form and can be secured in the microfluidic reaction chamber by van der Waals forces.

A heating element 150 is immediately adjacent and thermally coupled to the microfluidic reaction chamber 110 to heat a fluid when introduced therein. The heating element can be located over the substrate 120 and adjacent to the microfluidic reaction chamber, or the heating element can be recessed in a portion of the substrate, or both. The types of heating elements that can be used to include any on-board heating element that can be included in the rapid thermal cycling device structure. Examples can include a resistive heating element, a field-effect transistor, a p-n junction diode, a thin film heater, a thermal diode, or a combination thereof. In one example, the heating element can include a resistive heating element. In another example, the heating element can include a resistive heating element and a p-n junction diode. In yet another example, the heating element can include a resistive heating element and a thermistor. In some examples, there can be multiple heating elements, electrical contact pads, electrical traces, and the like. In one example, the heating element can be a system that can include multiple heating elements, feedback sensors, and a control loop operable to maintain a uniform temperature of a fluid when present in the microfluidic reaction chamber.

In some examples, materials of the heating element 150 can include platinum, aluminum, copper, gold, silver, tantalum, titanium, nickel, tin, zinc, chromium, tungsten silicon nitride, tungsten silicon nitride, tantalum nitride, tantalum silicide nitride, chromium silicon oxide, poly-silicon, germanium, conductive oxides (indium-tin oxides, zinc-tin oxide germanium-tin oxide and any other conductive and thermally stable oxides), tantalum-aluminum alloy, nichrome and other alloys, or a combination thereof. In one example, the heating element can include silver. In another example, the heating element can include poly-silicon. In yet another example, the heating element can include tungsten silicon nitride. In a further example, the heating element can include tantalum-aluminum alloy.

**The** heating element 150 can be dimensionally as large or larger in surface area as one of the surfaces defining the microfluidic reaction chamber 110, e.g., a floor surface, a side wall surface, a ceiling surface, etc. If the microfluidic reaction chamber is not box-like in shape, then the heating element can be as dimensionally large as the largest surface area of the microfluidic reaction chamber. Smaller dimensions for the heating element can likewise be used, but rapid thermal cycling can be enhanced when the fluid volume can be heated and cooled sufficiently throughout to cycle the full volume of the amplification fluid and/or more evenly distribute heat and cooling profiles for the fluid in the static microfluidic reaction chamber.

The heating element 150 can be operable to heat fluid when present in the microfluidic reaction chamber 110 at a rate of 100 °C/s to 50,000,000 °C/s (e.g., pulses of heat ranging in duration from the order of hundreds of nanoseconds to milliseconds), from 1,000 °C/s to 10,000,000 °C/s, or from 5,000 °C/s to 1,000,000 °C/s, for example. In one example, the heating element can be operable to heat a fluid when loaded in the microfluidic reaction chamber to a temperature ranging from 50 °C to 100 °C. The heating temperature can correlate to a temperature activated reaction. For example, a heating temperature for denaturing a double strand of a nucleic acid can range from 80 °C to 100 °C. A heating temperature for annealing a complementary nucleic acid sequence to a single strand of the nucleic acid can range from 50 °C to 65 °C. A heating temperature for extending the complementary nucleic acid sequence can range from 65 °C to 80 °C. The denaturing time, annealing time, and extending time can depend on the concentration of reagents and enzyme speeds. In one example, denaturing time, annealing time, and extending time can range from 0.002 second to 5 seconds, from 0.005 second to 5 seconds, from 0.05 second to 5 seconds, from 0.1 second to 1 second, from 1 second to 3 seconds, from 0.002 second to 1 second, or from 0.005 second to 0.5 second for the various temperature activated reactions. In one example, the heating element can be configured to cycle between temperatures for a specified time period. An example temperature cycle can include heating a fluid in the static microfluidic reaction chamber to a temperature ranging from 80 °C to 100 °C for 3 seconds, cooling to a temperature ranging from 50 °C to 65 °C for 1 to 2 seconds, and heating to a temperature ranging from 65 °C to 80 °C for 3 seconds.

The heating can be constant or pulsed. In one example, the heating can be pulsed. Pulsed heating can provide suitable control in heating a fluid in the microfluidic reaction chamber 110. In one example, the heating element 150 can be positioned to elevate a temperature of a fluid loaded in the microfluidic reaction chamber by 20 °C to 50 °C when pulsed on for 0.1 µs to 1 second and the substrate 120, the cover 130, or both in combination contribute to diffusion of heat from fluid loaded in the microfluidic reaction chamber in between heating element pulses. In some examples, the heating can occur at heating regions to permit even heat distribution throughout a fluid when present in the microfluidic reaction chamber.

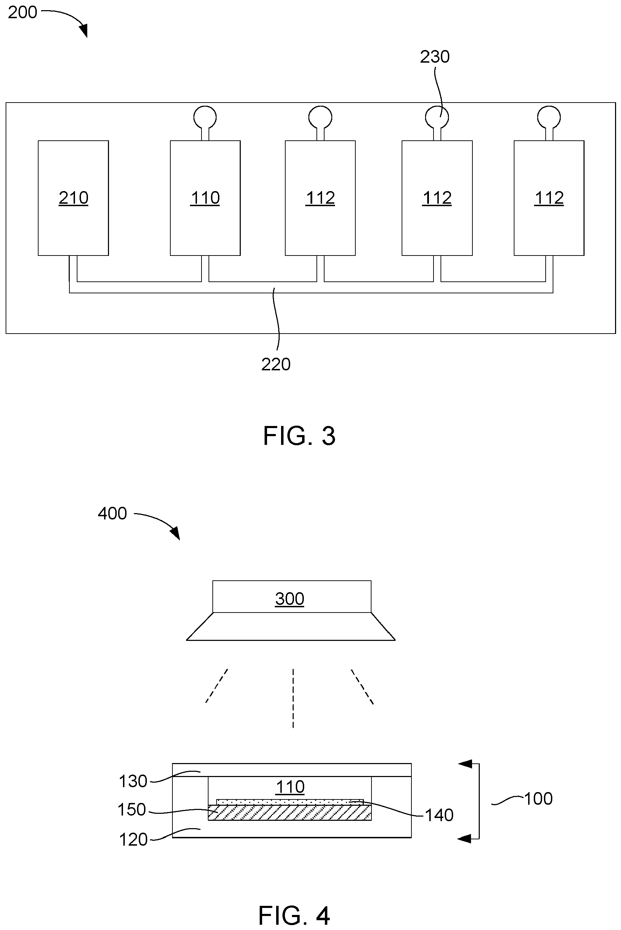

In some examples, as shown in FIG. 3, the rapid thermal cycling device 200 can be integrated in a microfluidic chip (as shown) that can include the microfluidic reaction chamber 110 (defined by the structures associated therewith as described in FIGS. 1A, 1B, and 2, for example). There can also be, however, additionally microfluidic reaction chambers, referred to herein collectively as secondary microfluidic reaction chambers 112. The term "secondary" does not infer secondary function or any type of use with respect to order or otherwise, but rather is a term of convenience to collectively name any number of additionally microfluidic chambers ranging from 2 to 100 microchambers, for example. In other examples, the number of microchambers is between 100 and 10,000. The additional microfluidic chambers can be arranged in parallel, in series, or a combination thereof. In one example, the rapid thermal cycling device can include a plurality of secondary microfluidic reaction chambers arranged fluidically in parallel with respect to the microfluidic reaction chamber. The plurality of secondary microfluidic reaction chambers can include one or more additional microfluidic reaction chambers. The secondary microfluidic reaction chambers can be similar to the microfluidic reaction chamber in that they can be made of similar materials, can have a similar size and shape, and can include the same dry reagent. In yet other examples, the additional reaction chambers can be different from the microfluidic reaction chamber in that they can differ from the microfluidic reaction chamber in one or more of the materials, size, shape, and/or dry reagent. In one example, each of the microfluidic reaction chamber can include a different dry reagent and can be operable to test for different nucleic acid sequences.

The plurality of microfluidic reaction chambers can be connected to a sample input microchannel 220 that can commonly feed the microfluidic reaction chamber and the plurality of the secondary microfluidic reaction chambers. Accordingly, the first microfluidic reaction chamber can receive a sample fluid prior to the pluralities of microfluidic reaction chambers, and so on. In one example, the sample input microchannel can have a linear and direct configuration. In yet other examples, the sample input microchannel can have a serpentine configuration. A serpentine configuration can increase the diffusion time between the microfluidic reaction chamber(s) filled with a sample fluid. The diffusion time between the microfluidic reaction chambers can be longer than the heating time for the sample fluid. In some examples a ratio of a width of the microfluidic reaction chamber to a length of the sample input microchannel can range from 1:3 to 1:100. In yet other examples a ratio of a width of the microfluidic reaction chamber to a length of the sample input microchannel can range from 1:3 to 1:10m, from 1:3 to 1:5, from 1:5 to 1:50, or from 1:25 to 1:75.

In one example, the microfluidic chip can further include a sample input port 210, air vents 230, or a combination thereof. The sample input port can be used to load a sample fluid therein. The air vent can allow air to escape the microfluidic chip as a sample is loaded into the chip through the sample input microchannel or the sample input port. In some examples, the microfluidic chip can have an N x M array configuration of microfluidic reaction chambers. In one example, the microfluidic chip can be an *in vitro* diagnostic point of care device. The microfluidic chip can include microfluidic channel, microfluidic chamber, circuit component, actuation mechanism, sensing mechanism, temperature controller, detector, or any combinations thereof.

The rapid thermal cycling device can be used to rapidly amplify a nucleic acid on time scale limits imposed by physical and chemical kinetics. For example, a balance of reaction and diffusion kinetics can be present where small devices on the scale described herein can provide a temporal response capable of letting the reaction kinetics of the chemistry of the amplification fluid be a rate-limiting factor, rather than the device properties. In other examples, the rapid thermal cycling device properties may respond more slowly than the reaction kinetics, but even in those instances, the thermal cyclizing can be very fast.

The rapid thermal cycling device can be used to amplify a nucleic acid within hold times from 0.05 seconds to 10 seconds, from 0.05 seconds to 1 second, from 0.5 seconds to 10 seconds, or from 0.5 seconds to 3 seconds for a denaturing, annealing, and extending phase during the amplification process. The rapid thermal cycling device can be used for polymerase chain reaction, isothermal amplification, loop mediated reverse transcription, forward transcription, or a combination thereof. The heating phase and cooling phase can follow rapid thermal cycling kinetics. In one example, the heating phase can include generating heating pulses from the heating element 150 lasting from 0.1 µs to 1 second. A full cycle of the thermally cycling which includes the heating phase and the cooling phase can occur at from about 1 millisecond to about 11 seconds, about 10 milliseconds to about 10 seconds, or about 100 milliseconds to about 5 seconds.

The rapid thermal cycling device can be used to amplify a nucleic acid sample that can include mRNA, RNA, DNA, or a combination thereof. In one example, the fluid sample can be a PCR ready fluid sample. Following amplification, the amplified nucleic acid can be used to diagnose a medical condition and/or detect the presence of a disease, or a pathogen. In some examples, the device can be incorporated in a lab on chip device that can be used in point of care diagnose of gonococcal infections, neisserial infections, trachomatis infections, *Mycobacterium tuberculosis*, *Neisseria gonorrhea*, *Chlamydia trachomatis*, malaria, HIV, AIDS, or any combinations thereof. Furthermore, the devices and systems described herein can also be used to amplify anything else with DNA or RNA using PCR amplification, for example, e.g., forensics, pathogen detection beyond human disease, anti-counterfeiting, etc. Furthermore, the capability of this type of nucleic acid amplification can be applied in the area of nucleic acid amplification testing (NAAT), which may include pathogen detection for infectious diseases, detection of foodborne pathogens, host response, or other epigenetic impact. In cancer testing, for example, the technique can be used to detect genetic mutations such as BRAF or KRAF. In essence, the devices and systems described herein can be applied to applications where amplification is use, such as in the case of concentrating nucleic acids prior to sequencing, e.g., DNA sequencing. In some examples, the diagnosis, detection, or some other similar function utilizing amplification as described herein can occur within 20 minutes of loading a nucleic acid sample into the rapid thermal cycling device. In some examples, the diagnosis can occur within 30 seconds to 20 minutes, 1 minute to 20 minutes, 1 minutes to 10 minutes, 1 minute to 5 minutes, 2 minutes to 20 minutes, 2 minutes to 10 minutes, 2 minutes to 5 minutes, etc., of loading a nucleic acid sample into the rapid thermal cycling device.

In further detail, a rapid thermal cycling system 400 is shown in FIG. 4. In this example, the system includes a rapid thermal cycling device 100 and a detection device 300. The rapid thermal cycling device includes a microfluidic reaction chamber 110 defined between a substrate 120 and a cover 130 having an average space therebetween from 4 µm to 150 µm; a dry reagent 140 positioned within the microfluidic reaction chamber, and a heating element 150 immediately adjacent and thermally coupled to the microfluidic reaction chamber to heat a fluid when present therein. Other structural features can also be present as shown in described in FIGS. 1A-3 previously. The detection device can be coupled to the microfluidic reaction chamber to receive data related to the fluid when present therein prior to, during, or after heat cycling of the fluid within the microfluidic reaction chamber. For example, the detecting device can be a device that detects interactions within the microfluidic reaction chamber optically, thermally, chemically, electrically, electrochemically, by gel-electrophoresis, or by a combination of detection technologies thereof. The rapid thermal cycling device can be as described above and can include any of the structural elements or combination of structural elements described above. In one example, the detection device can be a multi-color illumination and detection imaging system, an electrochemical detection system, an optical photodiode, or a combination thereof. In some examples, the rapid thermal cycling system can further include a remote thermal monitoring system to monitor a temperature of a fluid when present in the microfluidic reaction chamber and a feedback loop operable to adjust a temperature of the heating element and maintain a constant temperature of a fluid in the microfluidic reaction chamber.

Further presented herein is a method of manufacturing a rapid thermal cycling device. The method 500 includes loading 510 a reagent on a substrate, air drying or freeze-drying 520 the reagent on the substrate to form a dry reagent, and forming 530 a microfluidic chamber including a sample input port, wherein the microfluidic chamber is formed about the reagent or the dry reagent, the microfluidic chamber has an average height from 4 µm to 150 µm. Forming the microfluidic reaction chamber can occur either prior to or following loading of the reagent. In one example, the sample input port can be formed in the substrate prior to loading the reagent on the substrate. In another example, the sample input port can be formed after air drying or freeze drying. The sample input port can be formed for example, by laser machining the input port into a substrate or a cover. In some examples, the method can further include depositing a heating element on a substrate. In yet another example, the method can further include forming the microfluidic reaction chamber by applying a cover onto the substrate to leave the microfluidic reaction chamber therebetween, wherein forming the microfluidic reaction chamber occurs either prior to or after the air drying or freeze-drying.

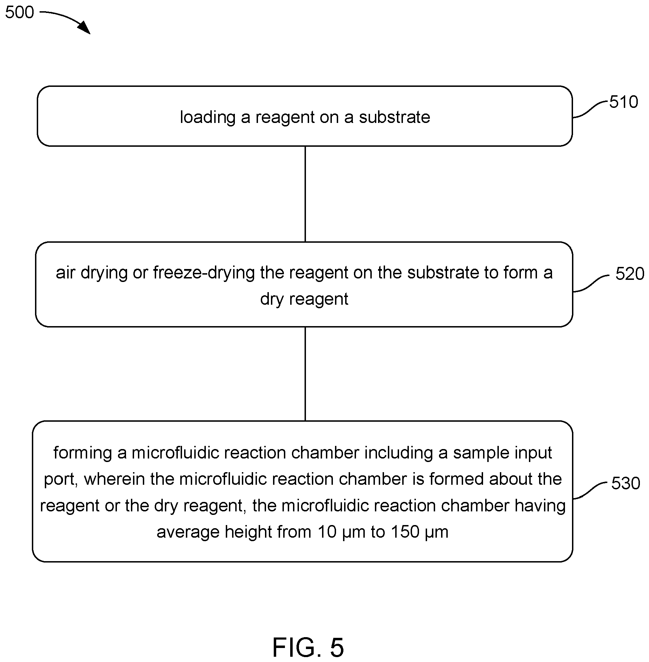

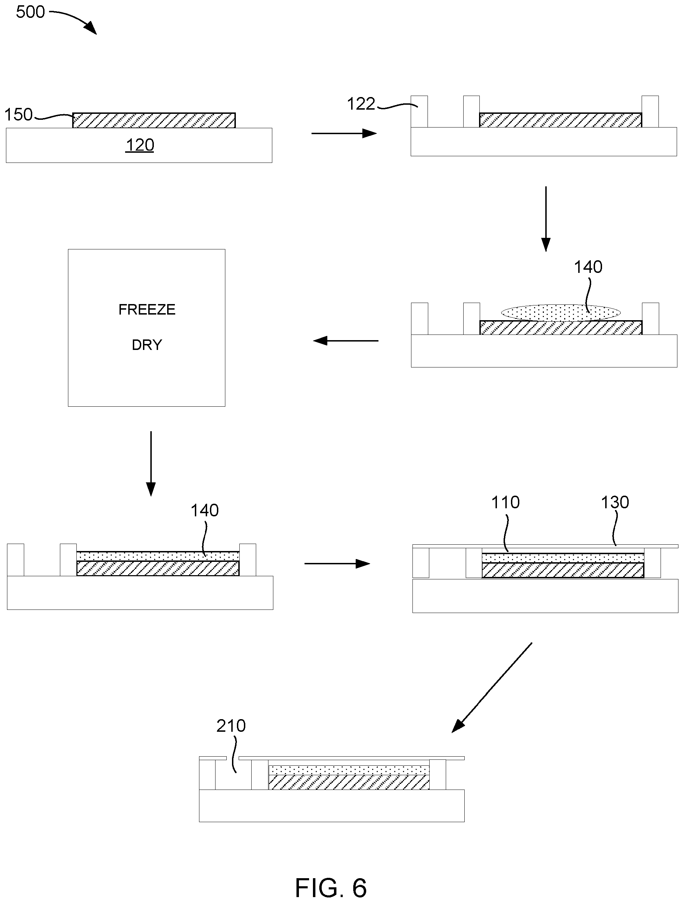

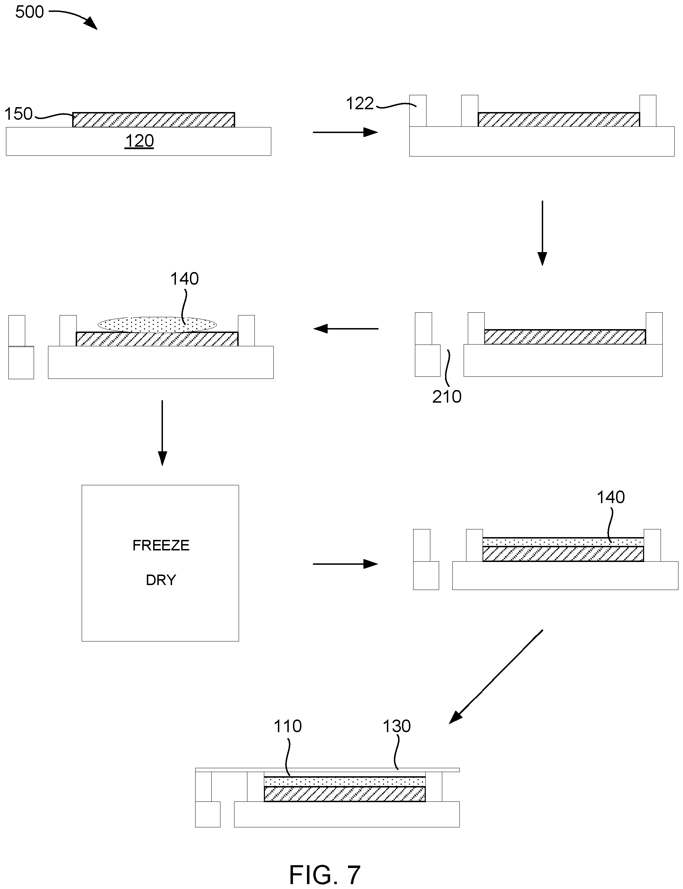

Figures 6 and 7 further graphically depict various methods of manufacturing 500 rapid thermal cycling devices for additional clarity. For ease of reference, the numerical reference for the structures in these figures is the same as the numerical reference used when referring to the method description (FIG. 5) the structural descriptions (FIGS. 1A-4) herein. In both of these figures a substrate 120 can have a heating element 150 included therein or thereon, and the substrate can include or be associated with supportive side walls 125. Notably, the sidewalls could alternatively be provided separately or provided by the cover 130. A liquid reagent 140 can be added and the device can be subsequently air dried or freeze-dried and the cover placed thereon. The microfluidic reaction chamber 110 can be defined by the space between the substrate, the side walls, and the cover. A sample input port 210 can be laser machined in the device either from above as depicted in FIG. 6 or from below as depicted in FIG. 7. The sample input port can be formed either following air drying or freeze-drying as depicted in FIG. 6 or prior to drying, such as by lyophilization, as depicted in FIG. 7. These and other methodologies can be implemented in accordance with examples herein.

It is noted that, as used in this specification and the appended claims, the singular forms "a," "an," and "the" include plural referents unless the content clearly dictates otherwise.

As used herein, a plurality of items, structural elements, compositional elements, and/or materials may be presented in a common list for convenience. However, these lists should be construed as though the individual member of the list is identified as a separate and unique member. Thus, no individual member of such list should be construed as a *de facto* equivalent of any other member of the same list based on the presentation in a common group without indications to the contrary.

Concentrations, dimensions, amounts, and other numerical data may be presented herein in a range format. A range format is used merely for convenience and brevity and should be interpreted flexibly to include the numerical values explicitly recited as the limits of the range, as well as to include all the individual numerical values or sub-ranges encompassed within that range as the individual numerical value and/or sub-range is explicitly recited. For example, a range of about 1 to about 20 should be interpreted to include the explicitly recited limits of 1 and 20 and individual value ranges therebetween, such as 1 to 10, 5 to 15, 10 to 20, etc. The invention is limited by the scope of the following claims.

## Claims

1. A rapid thermal cycling device (100, 200), comprising
a microfluidic reaction chamber (110) defined between a substrate (120) and a cover (130);
a dry reagent (140) positioned within the microfluidic reaction chamber; and
a heating element (150) thermally coupled to the microfluidic reaction chamber to heat a fluid when introduced therein,
wherein the substrate includes a thermal diffusing layer (122) has a thermal conductivity that ranges from 20 W/m/K to 2,000 W/m/K; and
**characterized in that**
the heating element is immediately adjacent to the microfluidic reaction chamber;
the microfluidic reaction chamber has an average space between the substrate and the cover from 4 µm to 150 µm; and
the thermal diffusing layer has a thickness that is 3 to 40 times greater than the average space between the substrate and the cover.

2. The rapid thermal cycling device of claim 1, wherein the heating element is located over the substrate.

3. The rapid thermal cycling device of claim 1, wherein the heating element is recessed in a portion of the substrate.

4. The rapid thermal cycling device of claim 1, wherein the substrate further comprises a thermal stabilizing layer (180) that is positioned and operable as a heat sink for the thermal diffusing layer.

5. The rapid thermal cycling device of claim 1, wherein a wall of the microfluidic chamber includes nucleic acid primers conjugated thereto.

6. The rapid thermal cycling device of claim 1, wherein the dry reagent includes thermostable nucleic acid polymerase, dNTPs, PCR primers, magnesium salt, or a combination thereof.

7. The rapid thermal cycling device of claim 1, wherein the heating element is thermally coupled the microfluidic reaction chamber to heat fluid in the microfluidic reaction chamber at a rate of 100 °C/s to 50,000,000 °C/s and wherein the heating element includes a heating interface surface that is dimensionally as large or larger in surface area as a microfluidic reaction chamber interface area where the substrate defines the microfluidic reaction chamber.

8. The rapid thermal cycling device of claim 1, further comprising a plurality of secondary microfluidic reaction chambers (112) arranged fluidically in parallel with respect to the microfluidic reaction chamber, wherein a sample input microchannel (220) commonly feeds the microfluidic reaction chamber and the plurality of secondary microfluidic reaction chambers.

9. The rapid thermal cycling device of claim 8, wherein a ratio of a width of the microfluidic reaction chamber to a length of the sample input microchannel ranges from 1:3 to 1:100.

10. The rapid thermal cycling device of claim 8, wherein the microfluidic channel has a serpentine configuration.

11. The rapid thermal cycling device of claim 1, wherein the microfluidic reaction chamber is included as part of an on-chip, internally controlled, device.

12. A rapid thermal cycling system, comprising:
a rapid thermal cycling device according to claim 1; and
a detection device (300) coupled to the microfluidic reaction chamber to receive data related to fluid prior to, during, or after heat cycling the fluid within the microfluidic reaction chamber.

13. The rapid thermal cycling system of claim 12, wherein the detection device includes a single-color illumination and detection imaging system, multi-color illumination and detection imaging system, an electrochemical detection system, an optical photodiode, or a combination thereof.

14. A method of manufacturing a rapid thermal cycling device, comprising:
loading (510) a reagent on a substrate;
air drying or freeze-drying (520) the reagent on the substrate to form a dry reagent; and
forming (530) a microfluidic reaction chamber including a sample input port, wherein the microfluidic reaction chamber is formed about the reagent or the dry reagent,
wherein the substrate includes a thermal diffusing layer (122) having a thermal conductivity that ranges from 20 W/m/K to 2,000 W/m/K; and
**characterized in that** the microfluidic reaction chamber has an average height from 4 µm to 150 µm;
the heating element is immediately adjacent to the microfluidic reaction chamber; and
the thermal diffusing layer has a thickness that is 3 to 40 times greater than the average space between the substrate and the cover.

15. The method of claim 13, further comprising forming the microfluidic reaction chamber by applying a cover onto the substrate to leave the microfluidic reaction chamber therebetween, wherein forming the microfluidic reaction chamber occurs either prior to or after the air drying or freeze-drying.

## Patentansprüche

1. Schnelltemperaturwechselbeanspruchungsvorrichtung (100, 200), die umfasst
eine mikrofluidische Reaktionskammer (110), die zwischen einem Substrat (120) und einer Abdeckung (130) definiert ist;
ein Trockenreagens (140), das innerhalb der mikrofluidischen Reaktionskammer positioniert ist; und
ein Heizelement (150), das mit der mikrofluidischen Reaktionskammer thermisch gekoppelt ist, um ein Fluid zu erhitzen, wenn es darin eingeführt wird,
wobei das Substrat eine thermische Diffusionsschicht (122) einschließt, die eine thermische Leitfähigkeit aufweist, die von 20 W/m/K bis 2.000 W/m/K reicht; und
**dadurch gekennzeichnet, dass**
das Heizelement unmittelbar an die mikrofluidische Reaktionskammer angrenzt;
die mikrofluidische Reaktionskammer einen durchschnittlichen Raum zwischen dem Substrat und der Abdeckung von 4 µm bis 150 µm aufweist; und
die thermische Diffusionsschicht eine Dicke aufweist, die 3 bis 40 Mal größer ist als der durchschnittliche Raum zwischen dem Substrat und der Abdeckung.

2. Schnelltemperaturwechselbeanspruchungsvorrichtung nach Anspruch 1, wobei sich das Heizelement über dem Substrat befindet.

3. Schnelltemperaturwechselbeanspruchungsvorrichtung nach Anspruch 1, wobei das Heizelement in einem Abschnitt des Substrats vertieft ist.

4. Schnelltemperaturwechselbeanspruchungsvorrichtung nach Anspruch 1, wobei das Substrat ferner eine thermische Stabilisierungsschicht (180) umfasst, die als ein Kühlkörper für die thermische Diffusionsschicht positioniert und betriebsfähig ist.

5. Schnelltemperaturwechselbeanspruchungsvorrichtung nach Anspruch 1, wobei eine Wand der mikrofluidischen Kammer Nukleinsäureprimer einschließt, die daran konjugiert sind.

6. Schnelltemperaturwechselbeanspruchungsvorrichtung nach Anspruch 1, wobei das Trockenreagens eine thermostabile Nukleinsäurepolymerase, dNTPs, PCR-Primer, Magnesiumsalz oder eine Kombination davon einschließt.

7. Schnelltemperaturwechselbeanspruchungsvorrichtung nach Anspruch 1, wobei das Heizelement mit der mikrofluidischen Reaktionskammer thermisch gekoppelt ist, um Fluid in der mikrofluidischen Reaktionskammer mit einer Geschwindigkeit von 100 °C/s bis 50.000.000 °C/s zu erhitzen, und wobei das Heizelement eine Heizschnittstellenoberfläche einschließt, die in dem Oberflächenbereich so groß wie oder größer als ein mikrofluidischer Reaktionskammerschnittstellenbereich ist, in dem das Substrat die mikrofluidische Reaktionskammer definiert.

8. Schnelltemperaturwechselbeanspruchungsvorrichtung nach Anspruch 1, ferner umfassend eine Vielzahl von sekundären mikrofluidischen Reaktionskammern (112), die parallel bezüglich der mikrofluidischen Reaktionskammer fluidisch angeordnet sind, wobei ein Probeneingangsmikrokanal (220) üblicherweise die mikrofluidische Reaktionskammer und die Vielzahl von sekundären mikrofluidischen Reaktionskammern speist.

9. Schnelltemperaturwechselbeanspruchungsvorrichtung nach Anspruch 8, wobei ein Verhältnis einer Breite der mikrofluidischen Reaktionskammer zu einer Länge des Probeneingangsmikrokanals von 1 : 3 bis 1 : 100 reicht.

10. Schnelltemperaturwechselbeanspruchungsvorrichtung nach Anspruch 8, wobei der mikrofluidische Kanal eine gewundene Konfiguration aufweist.

11. Schnelltemperaturwechselbeanspruchungsvorrichtung nach Anspruch 1, wobei die mikrofluidische Reaktionskammer als Teil einer intern gesteuerten On-Chip-Vorrichtung eingeschlossen ist.

12. Schnelltemperaturwechselbeanspruchungssystem, das umfasst:
eine Schnelltemperaturwechselbeanspruchungsvorrichtung nach Anspruch 1; und
eine Erfassungsvorrichtung (300), die mit der mikrofluidischen Reaktionskammer gekoppelt ist, um Daten, die sich auf Fluid beziehen, vor, während oder nach dem Erhitzen des Fluids innerhalb der mikrofluidischen Reaktionskammer zu empfangen.

13. Schnelltemperaturwechselbeanspruchungssystem nach Anspruch 12, wobei die Erfassungsvorrichtung ein Einfarbbeleuchtungs- und Erfassungsabbildungssystem, ein Mehrfarbbeleuchtungs-
und -Erfassungsabbildungssystem, ein elektrochemisches Erfassungssystem, eine optische Fotodiode oder eine Kombination davon einschließt.

14. Verfahren zum Herstellen einer Schnelltemperaturwechselbeanspruchungsvorrichtung, das umfasst:
Laden (510) eines Reagens auf ein Substrat;
Lufttrocknen oder Gefriertrocknen (520) des Reagens auf dem Substrat, um ein Trockenreagens auszubilden; und
Ausbilden (530) einer mikrofluidischen Reaktionskammer, die einen Probeneingangsanschluss einschließt, wobei die mikrofluidische Reaktionskammer um das Reagens oder das Trockenreagens ausgebildet wird,
wobei das Substrat eine thermische Diffusionsschicht (122) einschließt, die eine thermische Leitfähigkeit aufweist, die von 20 W/m/K bis 2.000 W/m/K reicht; und
**dadurch gekennzeichnet, dass** die mikrofluidische Reaktionskammer eine durchschnittliche Höhe von 4 µm bis 150 µm aufweist;
das Heizelement unmittelbar an die mikrofluidische Reaktionskammer angrenzt; und
die thermische Diffusionsschicht eine Dicke aufweist, die 3 bis 40 Mal größer ist als der durchschnittliche Raum zwischen dem Substrat und der Abdeckung.

15. Verfahren nach Anspruch 13, das ferner ein Ausbilden der mikrofluidischen Reaktionskammer durch Aufbringen einer Abdeckung auf das Substrat umfasst, um die mikrofluidische Reaktionskammer dazwischen zu verlassen, wobei ein Ausbilden der mikrofluidischen Reaktionskammer entweder vor oder nach dem Lufttrocknen oder dem Gefriertrocknen erfolgt.

## Revendications

1. Dispositif de cyclage thermique rapide (100, 200) comprenant
une chambre de réaction microfluidique (110) définie entre un substrat (120) et un couvercle (130) ;
un réactif sec (140) positionné à l'intérieur de la chambre de réaction microfluidique ; et
un élément chauffant (150) couplé thermiquement à la chambre de réaction microfluidique afin de chauffer un fluide lorsqu'il est introduit dans celle-ci,
dans lequel le substrat comporte une couche de diffusion thermique (122) ayant une conductivité thermique qui varie de 20 W/m/K à 2 000 W/m/K ; et
**caractérisé en ce que**
l'élément chauffant est immédiatement adjacent à la chambre de réaction microfluidique ;
la chambre de réaction microfluidique a un espace moyen entre le substrat et le couvercle de 4 µm à 150 µm ; et
la couche de diffusion thermique a une épaisseur qui est de 3 à 40 fois supérieure à l'espace moyen entre le substrat et le couvercle.

2. Dispositif de cyclage thermique rapide selon la revendication 1, dans lequel l'élément chauffant est situé au-dessus du substrat.

3. Dispositif de cyclage thermique rapide selon la revendication 1, dans lequel l'élément chauffant est encastré dans une partie du substrat.

4. Dispositif de cyclage thermique rapide selon la revendication 1, dans lequel le substrat comprend en outre une couche de stabilisation thermique (180) qui est positionnée et peut fonctionner comme un dissipateur de chaleur pour la couche de diffusion thermique.

5. Dispositif de cyclage thermique rapide selon la revendication 1, dans lequel une paroi de la chambre microfluidique comporte des amorces d'acide nucléique conjuguées à celle-ci.

6. Dispositif de cyclage thermique rapide selon la revendication 1, dans lequel le réactif sec comporte une polymérase d'acide nucléique thermostable, des dNTP, des amorces PCR, un sel de magnésium ou une combinaison de ceux-ci.

7. Dispositif de cyclage thermique rapide selon la revendication 1, dans lequel l'élément chauffant est couplé thermiquement à la chambre de réaction microfluidique afin de chauffer un fluide dans la chambre de réaction microfluidique à une vitesse de 100 °C/s à 50 000 000 °C/s et dans lequel l'élément chauffant comporte une surface d'interface chauffante qui est dimensionnellement aussi grande ou plus grande en surface que la zone d'interface de la chambre de réaction microfluidique où le substrat définit la chambre de réaction microfluidique.

8. Dispositif de cyclage thermique rapide selon la revendication 1, comprenant en outre une pluralité de chambres de réaction microfluidiques secondaires (112) disposées fluidiquement en parallèle par rapport à la chambre de réaction microfluidique, dans lequel un microcanal d'entrée d'échantillon (220) alimente communément la chambre de réaction microfluidique et la pluralité de chambres de réaction microfluidiques secondaires.

9. Dispositif de cyclage thermique rapide selon la revendication 8, dans lequel le rapport entre la largeur de la chambre de réaction microfluidique et la longueur du microcanal d'entrée d'échantillon varie de 1:3 à 1:100.

10. Dispositif de cyclage thermique rapide selon la revendication 8, dans lequel le canal microfluidique a une configuration en serpentin.

11. Dispositif de cyclage thermique rapide selon la revendication 1, dans lequel la chambre de réaction microfluidique est incluse en tant que partie d'un dispositif sur puce à commande interne.

12. Système de cyclage thermique rapide, comprenant :
un dispositif de cyclage thermique rapide selon la revendication 1 ; et
un dispositif de détection (300) couplé à la chambre de réaction microfluidique afin de recevoir des données relatives au fluide avant, pendant ou après un cyclage thermique du fluide à l'intérieur de la chambre de réaction microfluidique.

13. Système de cyclage thermique rapide selon la revendication 12, dans lequel le dispositif de détection comporte un système d'imagerie monochrome à éclairage et à détection, un système d'imagerie polychrome à éclairage et à détection, un système de détection électrochimique, une photodiode optique, ou une combinaison de ceux-ci.

14. Procédé de fabrication d'un dispositif de cyclage thermique rapide, comprenant :
le chargement (510) d'un réactif sur un substrat ;
le séchage à l'air ou la lyophilisation (520) du réactif sur le substrat afin de former un réactif sec ; et
la formation (530) d'une chambre de réaction microfluidique comportant un port d'entrée d'échantillon, dans lequel la chambre de réaction microfluidique est formée autour du réactif ou du réactif sec,
dans lequel le substrat comporte une couche de diffusion thermique (122) ayant une conductivité thermique qui varie de 20 W/m/K à 2 000 W/m/K ; et
**caractérisé en ce que** la chambre de réaction microfluidique a une hauteur moyenne de 4 µm à 150 µm ;
l'élément chauffant est immédiatement adjacent à la chambre de réaction microfluidique ; et
la couche de diffusion thermique a une épaisseur qui est de 3 à 40 fois supérieure à l'espace moyen entre le substrat et le couvercle.

15. Procédé selon la revendication 13, comprenant en outre la formation de la chambre de réaction microfluidique par application d'un couvercle sur le substrat afin de laisser la chambre de réaction microfluidique entre ceux-ci, dans lequel la formation de la chambre de réaction microfluidique a lieu soit avant soit après le séchage à l'air ou la lyophilisation.
